# EUROPEAN PATENT SPECIFICATION

(11) **EP 3 821 831 B1**
(45) Date of publication and mention of the grant of the patent: **16.04.2025**
(21) Application number: 19859023.4
(22) Date of filing: 04.09.2019
(51) Int. Cl.: A61B 17/221

(54) **CALCULUS REMOVING MESH BASKET AND DOUBLE-CAVITY END CAP FOR CALCULUS REMOVING MESH BASKET**
STEINENTFERNUNGSNETZKORB UND DOPPELKAVITÄTSENDKAPPE FÜR STEINENTFERNUNGSNETZKORB
PANIER MAILLÉ DE RETRAIT DE CALCUL ET CAPUCHON D'EXTRÉMITÉ À DOUBLE CAVITÉ POUR PANIER MAILLÉ DE RETRAIT DE CALCUL

(30) Priority: 11.09.2018 CN 201821481534 U
(43) Date of publication of application: 19.05.2021
(73) Proprietor: Micro-Tech (Nanjing) Co., Ltd., Nanjing, Jiangsu 210032 (CN)
(72) Inventor: XU, Chaowei, Nanjing, Jiangsu 210032 (CN); LI, Xiaochun, Nanjing, Jiangsu 210032 (CN); LI, Changqing, Nanjing, Jiangsu 210032 (CN); LENG, Derong, Nanjing, Jiangsu 210032 (CN); LIU, Chunjun, Nanjing, Jiangsu 210032 (CN)
(74) Representative: Patentanwaltskanzlei WILHELM & BECK
(86) International application number: PCT/CN2019/104414
(87) International publication number: WO 2020/052484

(56) References cited:
- EP-A1- 2 335 748
- WO-A1-2017/199066
- CN-A- 101 919 725
- CN-U- 202 699 229
- CN-U- 202 699 229
- CN-U- 204 133 542
- CN-U- 205 268 236
- US-A1- 2002 091 394
- US-A1- 2004 236 351
- US-A1- 2015 011 834
- US-B1- 6 440 061

## Description

### CROSS-REFERENCE TO RELATED DISCLOSURE

The present application claims the priority of Chinese patent application No. 201821481534.0, entitled "STONE EXTRACTION BASKET", submitted to the China Patent Office on September 11, 2018.

### FIELD OF INVENTION

The application relates to a stone extraction basket and a double lumen end cap for a stone extraction basket.

### BACKGROUND

Endoscopic Retrograde Cholangio Pancreatography (ERCP) refers to the technique of inserting the duodenoscope into the descending part of the duodenum, finding the duodenal papilla, inserting the radiographic catheter into the opening part of the papilla through the biopsy tube, injecting contrast agent and then taking X-ray photograph to show the pancreatic duct. Because ERCP does not require invasive surgery, trauma is small, and operation time is short, the complications are less than invasive surgery, hospitalization time is greatly shortened, and the ERCP is widely welcomed by patients. The stone extraction basket is used as a common calculus removing tool in ERCP fittings for taking out biliary stone. The stone extraction basket is divided into a guide wire mesh basket, a guide wire-free mesh basket and an integrated calculus removing and crushing mesh basket. According to the shape of the mesh basket, the stone extraction basket can be divided into a hexagonal shape mesh basket, a diamond shape mesh basket and a spiral shape mesh basket.

In traditional ERCP, instrument exchange is performed through the reserved long guide wire (4.5 m) in the human body, which requires cooperation by doctors and nurses and requires a high degree of proficiency in the cooperation of doctors and nurses, and the operation time is long. In order to save the operation time and achieve single-person operation by the doctor, a set of surgical instruments that can cooperate with a short guide wire (2.6 m) to achieve rapid exchange between ERCP instruments have been developed for ERCP. At present, the conventional stone extraction basket cannot be rapidly exchanged in ERCP with a short guide wire because of the lack of guide wire lumen or a guide wire lumen channel being too long. The stone extraction basket cooperated with the short guide wire is still in a development stage, and, at present, there exist the problems that the inlet of the guide wire lumen is stressed, stretched and deformed, the guide wire is difficult to insert, the guide wire is driven by the distal end cap of the mesh basket to prevent the mesh basket from removing calculus and being withdrawn from biliary tracts and the like.

US 2002/091394 A1 teaches the features of the preamble of claim 1. US 2002/091394 A1 discloses a stone extraction basket, comprising a mesh basket, an outer tube and an operation portion. The mesh basket is connected to the operation portion. The operation portion controls the mesh basket to move. A proximal end of the outer tube being connected to the operation portion. The outer tube axially extends from the proximal end to a distal end, the distal end of the outer tube being provided with a sidecar. A guide wire passes through one lumen channel of the sidecar. The mesh basket passes through the other lumen channel of the sidecar The sidecar has a connection portion to connect to the outer tube. Further prior art is disclosed by US20040236351A1, CN202699229U and US20150011834A1.

### SUMMARY OF THE INVENTION

The invention is defined by the independent claim. Preferred embodiments are disclosed in the subclaims.

The disclosure provides a stone extraction basket, comprising a mesh basket, an outer tube and an operation portion, the mesh basket being connected to the operation portion, the operation portion controlling the mesh basket to move, a proximal end of the outer tube being connected to the operation portion, the outer tube axially extending from the proximal end to a distal end, the distal end of the outer tube being provided with a double lumen end cap, a guide wire passing through one lumen channel of the double lumen end cap, and the mesh basket passing through the other lumen channel of the double lumen end cap.

Optionally, the operation portion comprises a handle and a core rod, the handle sliding back and forth along the core rod to extend or retract the mesh basket.

The double lumen end cap comprises a connection portion and a double lumen portion.

Optionally, the lumen channels of the double lumen end cap are hollow structures that together with the outer tube form a lumen channel through which the mesh basket is extended and retracted.

An outer surface of one end of the connection portion has a spiral barb structure for connection with the outer tube, and an outer surface of the other end of the connection portion has a protrusion structure for connection with the double lumen portion.

Optionally, the protrusion structure has a groove structure around it.

Optionally, the protrusion structure is a quadrangle, hexagonal, or octagonal stop structure.

Optionally, the connection portion and the double lumen portion are connected in a plastic-coated mode, an adhesive mode or a shrinkage fit mode.

Optionally, a distal end surface of the double lumen end cap has a guide bevel structure on one side of the lumen channel through which the guide wire passes.

Optionally, an outer contour thickness of the lumen channel through which the mesh basket passes in the double lumen end cap is greater than an outer contour thickness of the lumen channel through which the guide wire passes.

The present disclosure further discloses a double lumen end cap for a stone extraction basket comprising a connection portion and a double lumen portion, wherein the double lumen portion comprises two lumen channels, a guide wire passes through one lumen channel of the double lumen portion, and the mesh basket passes through the other lumen channel of the double lumen portion.

An outer surface of one end of the connection portion has a spiral barb structure and an outer surface of the other end of the connection portion has a protrusion structure.

Optionally, the protrusion structure has a groove structure around it.

Optionally, a distal end surface of the double lumen portion has a guide bevel structure on one side of the lumen channel through which the guide wire passes.

Optionally, an outer contour thickness of the lumen channel through which the mesh basket passes in the double lumen portion is greater than an outer contour thickness of the lumen channel through which the guide wire passes.

The connection portion and the double lumen portion can be made of stainless steel or medical polymer materials.

Due to the fact that organs such as a biliary tract of a human body and the like are not tubular structures with regular shapes, the diameter of the outer tube is relatively large, and parts such as a mesh basket are also arranged in the outer tube, the flexibility is relatively poor in the using process. The outer tube can easily deviate from a preset line when the outer tube is pushed at a bent position. In the stone extraction basket of the present disclosure, the distal end of the outer tube is provided with a double lumen end cap, and the double lumen end cap is close to an inner wall of the outer tube. In use, the guide wire may be pushed to a predetermined position using a sphincterotome. When the stone extraction basket is required to be used for calculus removing, the guide wire can pass through the guide wire lumen in advance. The guide wire is fixed, the outer tube is pushed, the outer tube moves along the guide wire, and the outer tube can move according to a preset line.

### BRIEF DESCRIPTION OF THE DRAWINGS

In order to more clearly illustrate the technical solution of the present disclosure, the drawings used in the embodiments will be briefly described below, and it would be possible for a person skilled in the art to obtain other drawings according to these drawings without involving any inventive effort.
Fig. 1 is a general schematic view of a stone extraction basket;
Fig. 2A is a cross-sectional view of a double lumen end cap;
Fig. 2B is a schematic view of a structure of the double lumen end cap;
Fig. 3 is a schematic view of a head end connector of the double lumen end cap;
Fig. 4A is a schematic view of the stone extraction basket in determining the location of a calculus in a biliary tract;
Fig. 4B is a schematic view of the stone extraction basket when tightened;
Fig. 4C is a schematic view of the stone extraction basket removing the calculus;

List of reference numerals in the drawings:
1 mesh basket
2 outer tube
3 sheath tube
4 guide wire
5 double lumen end cap
6 lumen channel A
7 lumen channel B
8 connection portion
9 double lumen portion
10 operation portion
11 handle
12 core rod
13 Luer fitting
14 connector
15 calculus
16 endoscope
17 biliary tract
18 duodenal papilla
19 spiral barb structure
20 protrusion structure
21 groove structure

### DESCRIPTION OF THE EMBODIMENTS

For the purposes, technical solutions, and advantages of the present disclosure to become more fully apparent, the present disclosure would be further illustrated in detail in combination with the accompanying drawings and embodiments. It should be understood that the specific embodiments described herein are merely illustrative of the present disclosure and are not intended to be limiting thereof.

Hereinafter, an end close to the operator is defined as a proximal end, and an end far from the operator is defined as a distal end.

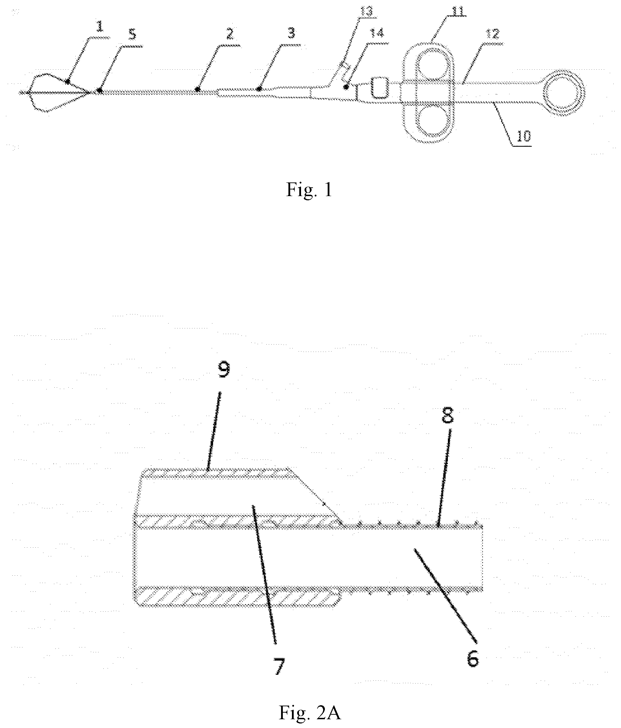

Fig. 1 is a general schematic view of a stone extraction basket. The stone extraction basket of the present disclosure comprises a mesh basket 1, an outer tube 2 and an operation portion 10. The mesh basket 1 is connected to the operation portion so that the operation portion 10 controls the mesh basket 1 to move. A proximal end of the outer tube 2 is connected to the operation portion, and the outer tube axially extends from the proximal end to a distal end along the stone extraction basket. The operation portion 10 comprises a handle 11 and a core rod 12, wherein the core rod 12 axially extends from the proximal end to the distal end along the stone extraction basket, the handle 11 is positioned at the proximal end of the stone extraction basket and is arranged on the core rod 12, and the handle 11 slides back and forth along the core rod 12 to extend or retract the mesh basket 1. The proximal end of the outer tube 2 is provided with a sheath tube 3, and a proximal end of the sheath tube 3 is connected to the core rod 12 via a connector 14. The distal end of the outer tube 2 of the stone extraction basket of the present disclosure is provided with a double lumen end cap 5.

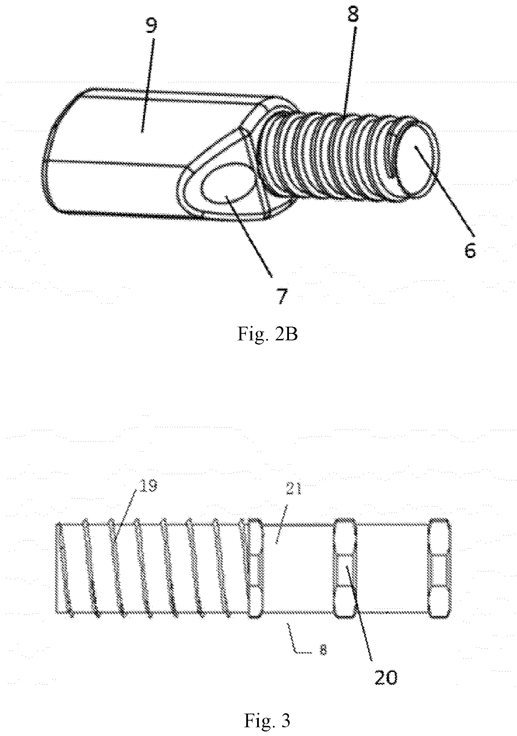

As shown in Figs. 1 and 2A -2B, the double lumen end cap 5 includes a connection portion 8 and a double lumen portion 9. The guide wire 4 passes through one lumen channel, i.e., lumen channel B7, of the double lumen end cap 5, and the mesh basket 1 passes through the other lumen channel, i.e. lumen channel A6, of the double lumen end cap 5. The two lumen channels of the double lumen end cap are hollow structures that together with the outer tube 2 form a lumen channel through which the mesh basket 1 is extended and retracted.

As shown in Fig. 3, one end of the connection portion 8 is connected with the double lumen portion 9, and the other end of the connection portion 8 is connected with the outer tube 2. One end of the connection portion 8 connected with the outer tube 2 is provided with a spiral barb structure 19 on an outer surface of the connection portion 8. One end of the connection portion 8 connected with one end of the double lumen portion 9 is provided with a protrusion structure 20 arranged on the outer surface of the connection portion 8. A groove structure 21 can be arranged around the protrusion structure 20, and the protrusion structure 20 and the groove structure 21 enable the connection portion 8 to be better connected with the double lumen portion 9. The connection portion 8 and the double lumen portion 9 may be connected in a plastic-coated mode, an adhesive mode, a shrinkage fit mode, or other mode. The protrusion structure 20 may be a quadrangle, hexagonal or octagonal stop structure. The double lumen end cap is arranged at the distal end of the stone extraction basket of the present disclosure, so that a guide wire can pass therethrough, the strength of the guide wire lumen is enhanced, deformation or breakage of the guide wire lumen is avoided, and interference with the guide wire after the mesh basket is opened is avoided.

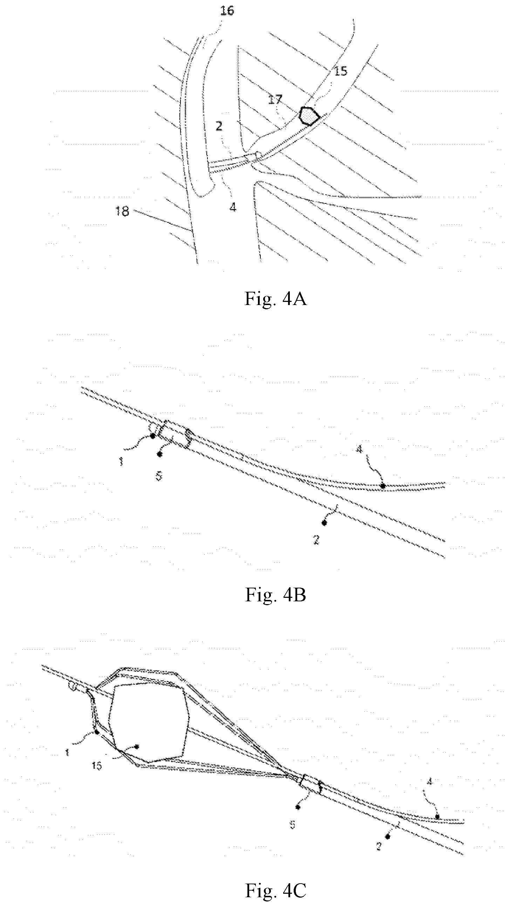

As shown in Figs. 1, 2B and 4A-4C, the stone extraction basket has a double lumen end cap 5, and during actual use, the guide wire 4 passes through the lumen channel B7 of the double lumen end cap 5 and the mesh basket 1 is placed in the lumen channel A6 of the double lumen end cap 5. The entire instrument is inserted along the guide wire 4 into an endoscopic channel, the outer tube 2 of the stone extraction basket is pushed to the duodenal papilla 18, the duodenal papilla 18 is viewed under the endoscope 16, and the distal end of the outer tube 2 of the stone extraction basket is inserted through the duodenal papilla 18 into the biliary tract 17. A syringe is used to inject contrast agent from the Luer fitting 13 of the stone extraction basket to assist in locating the mesh basket 1 and calculus 15. The outer tube 2 of the stone extraction basket is advanced distally over the calculus 15 intended to be captured. By pushing the handle 11 of the operation portion 10 towards the distal end, the mesh basket 1 is slowly opened, and calculus 15 is captured by pulling the mesh basket 1. After the mesh basket 1 captures the calculus 15, the handle 11 of the operation portion 10 is pulled back, and the calculus 15 is tightened and fixed on a distal end surface of the outer tube 2 of the stone extraction basket. The handle 11 is withdrawn proximally and the mesh basket 1 is withdrawn until the calculus 15 is pulled out of the biliary tract 17. If there are multiple calculi, the procedure is repeated until all calculi are removed.

In some embodiments of the present disclosure, the distal end surface of the double lumen end cap 5 has a guide bevel structure on one side of the lumen channel through which the guide wire 4 passes. The guide bevel is added to the distal end surface of the double lumen portion 9 of the double lumen end cap 5, so that the performance of the stone extraction basket passing through the duodenoscope can be improved. Therefore, interference between the double lumen end cap 5 and a forceps lifter at an outlet position at the distal end of the duodenoscope can be avoided. When a head end cap at the distal end of the catheter passes through the forceps lifter, the catheter can smoothly slide through the forceps lifter under the guiding effect of the bevel. In practice, if there is no guide bevel structure, the double lumen end cap 5 will first contact the forceps lifter through a lumen channel end surface of the guide wire to form a jam.

In addition, in practical use, an object taking operation is carried out after the stone extraction basket passes through the duodenoscope, so that the double lumen end cap 5 is likely to contact with an inner wall of tissues of organs such as biliary tracts or duodenums of a patient. Therefore, the guide bevel is increased on the distal end surface of the double lumen portion 9 of the double lumen end cap 5, the tissues can be dredged, the inner wall of the tissues is prevented from obstructing the action of the stone extraction basket, and the tissues are prevented from being injured due to the scratching by the end surface of the double lumen end cap 5.

In some embodiments of the present disclosure, an outer contour thickness of the lumen channel through which the mesh basket passes in the double lumen portion is greater than an outer contour thickness of the lumen channel through which the guide wire passes. In actual use, typically, a dimension of the part of the endoscopic instruments inserted into the human body is expressed in french, i.e., the circumference of the cross-section of the inserted part of the instruments. Because the natural lumen channel of the human body is in irregular peristalsis, when an instrument with a special-shaped cross section is inserted into the natural lumen channel of the human body, the smaller the circumference of the cross section, the better the insertion performance. The insertion performance of an instrument is a very important indicator for evaluating the performance of the instrument. Many times, very small channels in the human body, such as papilla openings, require smaller french and better insertion performance of the instrument to facilitate insertion and subsequent surgical procedures. Therefore, since the outer contour thickness of the lumen channel through which the mesh basket passes in the double lumen end cap is greater than the outer contour thickness of the lumen channel through which the guide wire passes, the dimension of the cross section of the catheter can be reduced as much as possible, better insertion performance is achieved, and the efficiency of inserting the stone extraction basket into the duodenal papilla is improved.

The foregoing is merely preferred embodiments of the present disclosure to enable a person skilled in the art to understand or practice the present disclosure. Various modifications and combinations of these embodiments will be readily apparent to those skilled in the art, and the generic principles defined herein may be implemented in other embodiments. Thus, the present disclosure is not intended to be limited to the embodiments shown herein but is to be accorded the widest scope consistent with the principles and novel features disclosed herein. The invention is defined by the appended claims.

## Claims

1. A stone extraction basket, comprising a mesh basket (1), an outer tube (2) and an operation portion (10), the mesh basket (1) being connected to the operation portion (10), the operation portion (10) controlling the mesh basket (1) to move, a proximal end of the outer tube (2) being connected to the operation portion (10), the outer tube (2) axially extending from the proximal end to a distal end, the distal end of the outer tube being provided with a double lumen end cap (5), a guide wire (4) passing through one lumen channel (9) of the double lumen end cap (5), the mesh basket (1) passing through the other lumen channel (6) of the double lumen end cap (5), wherein the double lumen end cap (5) comprises a connection portion (8) and a double lumen portion (9), the double lumen portion (9) comprising the one lumen channel (7) and the other lumen channel (6),
**characterized in that**
an outer surface of one end of the connection portion (8) has a spiral barb structure (19) for connection with the outer tube (2), and an outer surface of the other end of the connection portion has a protrusion structure (20) for connection with the double lumen portion (9).

2. The stone extraction basket according to claim 1, wherein the operation portion (10) comprises a handle (11) and a core rod (12), the handle (11) sliding back and forth along the core rod (12) to extend or retract the mesh basket (1).

3. The stone extraction basket according to claim 1 or 2, wherein the lumen channels (6, 7) of the double lumen end cap (5) are hollow structures that together with the outer tube (2) form a lumen channel through which the mesh basket (1) is extended and retracted.

4. The stone extraction basket according to any of claims 1 to 3, wherein the protrusion structure (20) has a groove structure (21) around it.

5. The stone extraction basket according to any of claims 1 to 4, wherein the protrusion structure (20) is a quadrangle, hexagonal, or octagonal stop structure.

6. The stone extraction basket according to any of claims 1 to 5, wherein the connection portion (8) and the double lumen portion (9) are connected in a plastic-coated mode, an adhesive mode or a shrinkage fit mode.

7. The stone extraction basket according to any of claims 1 to 6, wherein a distal end surface of the double lumen end cap (5) has a guide bevel structure on one side of the lumen channel through which the guide wire passes.

8. The stone extraction basket according to any of claims 1 to 7, wherein an outer contour thickness of the lumen channel through (6) which the mesh basket (1) passes in the double lumen end cap (5) is greater than an outer contour thickness of the lumen channel (7) through which the guide wire (4) passes.

## Patentansprüche

1. Steinentfernungsnetzkorb, umfassend einen Netzkorb (1), ein Außenrohr (2) und einen Bedienungsabschnitt (10), wobei der Netzkorb (1) mit dem Bedienungsabschnitt (10) verbunden ist, der Bedienungsabschnitt (10) den Netzkorb (1) zur Bewegung steuert, ein nahe gelegenes Ende des Außenrohrs (2) mit dem Bedienungsabschnitt (10) verbunden ist, das Außenrohr (2) sich axial vom nahe gelegenen Ende zu einem fern gelegenen Ende erstreckt, das fern gelegene Ende des Außenrohrs mit einer Doppellumenendkappe (5) versehen ist, ein Führungsdraht (4) durch einen Lumenkanal (9) der Doppellumenendkappe (5) verläuft, der Netzkorb (1) durch den anderen Lumenkanal (6) der Doppellumenendkappe (5) verläuft, wobei die Doppellumenendkappe (5) einen Verbindungsabschnitt (8) und einen Doppellumenabschnitt (9) umfasst, wobei der Doppellumenabschnitt (9) den einen Lumenkanal (7) und den anderen Lumenkanal (6) umfasst,
**dadurch gekennzeichnet, dass**
eine Außenfläche eines Endes des Verbindungsabschnitts (8) eine spiralförmige Widerhakenstruktur (19) zur Verbindung mit dem Außenrohr (2) aufweist und eine Außenfläche des anderen Endes des Verbindungsabschnitts eine Vorsprungsstruktur (20) zur Verbindung mit dem Doppellumenabschnitt (9) aufweist.

2. Steinentfernungsnetzkorb nach Anspruch 1, wobei der Bedienungsabschnitt (10) einen Griff (11) und eine Kernstange (12) umfasst, der Griff (11) entlang der Kernstange (12) hin- und hergleitet, um den Netzkorb (1) auszufahren oder einzuziehen.

3. Steinentfernungsnetzkorb nach Anspruch 1 oder 2, wobei die Lumenkanäle (6, 7) der Doppellumenendkappe (5) Hohlstrukturen sind, die zusammen mit dem Außenrohr (2) einen Lumenkanal bilden, durch den der Netzkorb (1) ausgefahren und eingezogen wird.

4. Steinentfernungsnetzkorb nach einem der Ansprüche 1 bis 3, wobei die Vorsprungsstruktur (20) eine diese umgebende Nutstruktur (21) aufweist.

5. Steinentfernungsnetzkorb nach einem der Ansprüche 1 bis 4, wobei die Vorsprungsstruktur (20) eine viereckige, sechseckige oder achteckige Anschlagstruktur ist.

6. Steinentfernungsnetzkorb nach einem der Ansprüche 1 bis 5, wobei der Verbindungsabschnitt (8) und der Doppellumenabschnitt (9) in einer kunststoffbeschichteten Weise, einer Klebeweise oder einer Schrumpfpassungsweise verbunden sind.

7. Steinentfernungsnetzkorb nach einem der Ansprüche 1 bis 6, wobei eine distale Endfläche der Doppellumenendkappe (5) eine Führungsabschrägungsstruktur auf einer Seite des Lumenkanals aufweist, durch den der Führungsdraht verläuft.

8. Steinentfernungsnetzkorb nach einem der Ansprüche 1 bis 7, wobei eine äußere Konturdicke des Lumenkanals (6), durch den der Netzkorb (1) in der Doppellumenendkappe (5) verläuft, größer als eine äußere Konturdicke des Lumenkanals (7) ist, durch den der Führungsdraht (4) verläuft.

## Revendications

1. Panier d'extraction de calcul, comprenant un panier maillé (1), un tube externe (2) et une portion d'actionnement (10), le panier maillé (1) étant raccordé à la portion d'actionnement (10), la portion d'actionnement (10) commandant le panier maillé (1) pour qu'il se déplace, une extrémité proximale du tube externe (2) étant raccordée à la portion d'actionnement (10), le tube externe (2) s'étendant axialement depuis l'extrémité proximale jusqu'à une extrémité distale, l'extrémité distale du tube externe étant pourvue d'un capuchon d'extrémité à double lumière (5), un fil de guidage (4) passant à travers un premier canal de lumière (9) du capuchon d'extrémité à double lumière (5), le panier maillé (1) passant à travers l'autre canal de lumière (6) du capuchon d'extrémité à double lumière (5), le capuchon d'extrémité à double lumière (5) comprenant une portion de raccordement (8) et une portion à double lumière (9), la portion à double lumière (9) comprenant le premier canal de lumière (7) et l'autre canal de lumière (6),
**caractérisé en ce que**
une surface externe d'une première extrémité de la portion de raccordement (8) a une structure barbelée en spirale (19) pour raccordement au tube externe (2), et une surface externe de l'autre extrémité de la portion de raccordement a une structure de saillie (20) pour raccordement à la portion à double lumière (9).

2. Panier d'extraction de calcul selon la revendication 1, dans lequel la portion d'actionnement (10) comprend une poignée (11) et une tige centrale (12), la poignée (11) coulissant en va-et-vient le long de la tige centrale (12) pour étendre ou rétracter le panier maillé (1).

3. Panier d'extraction de calcul selon la revendication 1 ou la revendication 2, dans lequel les canaux de lumière (6, 7) du capuchon d'extrémité à double lumière (5) sont des structures creuses qui, conjointement avec le tube externe (2), forment un canal de lumière à travers lequel le panier maillé (1) est étendu et rétracté.

4. Panier d'extraction de calcul selon l'une quelconque des revendications 1 à 3, dans lequel la structure de saillie (20) a une structure de rainure (21) autour d'elle.

5. Panier d'extraction de calcul selon l'une quelconque des revendications 1 à 4, dans lequel la structure de saillie (20) est une structure d'arrêt quadrilatérale, hexagonale ou octogonale.

6. Panier d'extraction de calcul selon l'une quelconque des revendications 1 à 5, dans lequel la portion de raccordement (8) et la portion à double lumière (9) sont raccordées dans un mode revêtu de plastique, un mode adhésif ou un mode d'ajustement fretté.

7. Panier d'extraction de calcul selon l'une quelconque des revendications 1 à 6, dans lequel une surface d'extrémité distale du capuchon d'extrémité à double lumière (5) a une structure de biseau de guidage sur un côté du canal de lumière à travers lequel le fil de guidage passe.

8. Panier d'extraction de calcul selon l'une quelconque des revendications 1 à 7, dans lequel une épaisseur de contour externe du canal de lumière à travers (6) lequel passe le panier maillé (1) dans le capuchon d'extrémité à double lumière (5) est supérieure à une épaisseur de contour externe du canal de lumière (7) à travers lequel le fil de guidage (4) passe.
